(19) Europäisches Patentamt

European Patent Office

Office européen des brevets

(11) Veröffentlichungsnummer: **0 251 248**
**A2**

(12) **EUROPÄISCHE PATENTANMELDUNG**

(21) Anmeldenummer: 87109235.9

(22) Anmeldetag: 26.06.87

(51) Int. Cl.⁴: **C11D 1/83** , A61K 7/08 ,
A61K 7/50

(30) Priorität: 04.07.86 DE 3622438

(43) Veröffentlichungstag der Anmeldung:
**07.01.88 Patentblatt 88/01**

(84) Benannte Vertragsstaaten:
**AT BE CH DE ES FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Hensen, Hermann, Dr.**
**Tizianweg 4**
**D-4010 Hilden(DE)**
Erfinder: **Stuhrmann, Dagmar**
**Eichelstrasse 62**
**D-4000 Düsseldorf(DE)**
Erfinder: **Lindner, Renate**
**Poststrasse 24**
**D-4010 Hilden(DE)**
Erfinder: **Kruse, Renate**
**Benrodestrasse 90 a**
**D-4000 Düsseldorf 13(DE)**

(54) **Kosmetischer Waschrohstoff.**

�疗 Ein gut hautverträglicher Waschrohstoff in Form eines fließfähigen Konzentrats enthält 0,4 bis 0,6 Mol/kg eines Alkylethersulfats auf Basis eines speziellen Gemisches von Fettalkoholoxethylaten, welches teils als Mg-Salz, teils als Na-Salz vorliegt, 0,03 bis 0,08 Mol/kg eines Ölsäure und/oder Linolsäurediethanolamids und 1,4 bis 1,6 Mol/kg Natriumionen in Form von Natriumchlorid und/oder Natriumsulfat. Das Produkt eignet sich zur Verwendung in kosmetischen Haar-und Körperwaschmitteln.

EP 0 251 248 A2

## "Kosmetischer Waschrohstoff"

Gegenstand der Erfindung ist ein neuer kosmetischer Waschrohstoff auf der Basis von Alkylethersulfattensiden, der durch einen Gehalt spezieller Fettsäurealkanolamide sowie Natrium-und Magnesium-Ionen besondere rheologische Eigenschaften aufweist.

Kosmetische Waschrohstoffe, die für die Herstellung von flüssigen Haar-und Körperreinigungsmitteln geeignet sind, müssen neben einem guten Reinigungsvermögen ein gutes Schäumvermögen und eine gute Haut-und Schleimhautverträglichkeit aufweisen. Darüber hinaus ist es von großem Vorteil, wenn ein solcher Rohstoff selbst bei hoher Konzentration noch flüssig und pumpfähig ist, aber nach Verdünnung auf Anwendungskonzentration nicht zu dünnflüssig wird, oder sich noch gut verdicken läßt, so daß das Reinigungsmittel bei der Anwendung nicht wasserdünn zwischen den Fingern hindurchrinnt.

Es war bekannt, daß sich Alkylethersulfate, besonders in Form ihrer Magnesiumsalze durch eine besonders gute Haut-und Schleimhautverträglichkeit auszeichnen. Dies gilt vor allem für Ethersulfate mit einem hohen Ethoxylierungsgrad. Leider nimmt aber das Schäumvermögen und die Viskosität und Verdickbarkeit der verdünnten Lösungen durch Elektrolyte mit steigendem Ethoxylierungsgrad stark ab. Es ist auch bekannt, mit Fettsäurealka nolamiden die Viskosität und Verdickbarkeit verdünnter Lösungen von Tensiden anzuheben. In Lösungen höherer Konzentration, wie sie für die Waschrohstoffe üblich und wirtschaftlich erforderlich sind, führt ein Zusatz von Fettsäurealkanolamiden jedoch leicht zur Gelbildung und zum Verlust der Pumpfähigkeit.

Es bestand daher die Aufgabe, einen kosmetischen Waschrohstoff mit hoher Haut-und Schleimhautverträglichkeit, guter Pumpfähigkeit und gutem Schäumvermögen zu finden, der auch nach Verdünnung auf einen Bruchteil der Ausgangskonzentration durch Elektrolytzusatz, z.B. durch Natriumchlorid-Zusatz wieder verdickt werden kann.

Es wurde gefunden, daß diese Anforderungen weitgehend erfüllt werden durch einen kosmetischen Waschrohstoff in Form eines bei 20 °C pumpbaren wässrigen Konzentrates, welches

0,4 - 0,6 Mol/kg eines Alkylethersulfatgemisches aus sulfatierten Ethoxylaten, die zusammengesetzt sind aus 35 bis 45 Gewichtsprozent eines Anlagerungsproduktes von 8 bis 12 Mol Ethylenoxid an einen im wesentlichen linearen $C_{12}$-$C_{18}$-Fettalkoholschnitt.

40 bis 60 Gewichtsprozent eines Anlagerungsproduktes von 1 bis 3 Mol Ethylenoxid an einen im wesentlichen linearen $C_{12}$-$C_{14}$-Fettalkoholschnitt.

0 bis 20 Gewichtsprozent eines Anlagerungsproduktes von 1 bis 3 Mol Ethylenoxid an ein Oleyl-Cetylalkohol-Gemisch.

wovon 0,2 bis 0,3 Mol als Magnesiumsalz und der Rest als Natriumsalz vorliegt,

0,03 - 0,08 Mol/kg Ölsäure-und/oder Linolsäurediethanolamid und

1,4 - 1,6 Mol/kg Natrium-ionen in Form von Natriumchlorid und/oder Natriumsulfat

und im übrigen im wesentlichen Wasser enthält. Der erfindungsgemäße Waschrohstoff zeichnet sich nicht nur durch eine besonders gute Schleimhautverträglichkeit und ein gutes Schäumvermögen aus, sondern steigt beim Verdünnen mit ca. 20 Teilen Wasser auf 100 Teile des Waschrohstoffs in der Viskosität auf den 3 bis 4-fachen Wert an. Wässrige Lösungen mit 10 Gewichtsprozent Aniontensid lassen sich durch Zusatz von Natriumchlorid auf Viskositäten von 5 bis 10 000 mPa s (20 °C) verdicken.

Die Herstellung des erfindungsgemäßen Waschrohstoffes erfolgt am einfachsten in der Weise, daß man zunächst die Ethoxylate vereinigt und die Hydroxylzahl (d.h. das Molekulargewicht) der Mischung ermittelt.

Das Anlagerungsprodukt von 8 bis 12 Mol Ethylenoxid an einen im wesentlichen linearen $C_{12}$-$C_{18}$-Fettalkoholschnitt wird nach bekannten Verfahren durch Ethoxylierung eines geeigneten Fettalkohols, z.B. eines Kokos-oder Palmkernfettalkoholschnittes mit 12 bis 18 C-Atomen hergestellt. Solche Alkoholschnitte aus Kokosfettalkohol enthalten z.B. 0 bis 3 Gewichtsprozent n-Decanol, 45 bis 60 Gewichtsprozent, n-Dodecanol, 15 bis 30 Gewichtsprozent n-Tetradecanol, 6 bis 15 Gewichtsprozent n-Hexadecanol und 10 bis 15 Gewichtsprozent n-Octadecanol.

In gleicher Weise wird das Anlagerungsprodukt von 1 bis 3 Mol Ethylenoxid an einen im wesentlichen linearen $C_{12}$-$C_{14}$-Fettalkoholschnitt z.B. ausgehend von einem Kokos-oder Palmkernfettalkoholschnitt mit 12 bis 14 C-Atomen hergestellt. Solche Fettalkoholschnitte enthalten z.B. 0 bis 2 Gewichtsprozent n-Decanol, 70 bis 75 Gewichtsprozent n-Dodecanol, 25 bis 30 Gewichtsprozent n-Tetradecanol und 0 bis 5 Gewichtsprozent n-Hexadecanol.

Anstelle der genannten gesättigten Kokos-oder Palmkernfettalkohole können auch synthetische, nicht oder wenig verzweigte Fettalkohole eingesetzt werden, wenn diese zu mindestens 90 Gewichtsprozent aus den spezifizierten C-Kettenlängen bestehen.

Das Anlagerungsprodukt von 1 bis 3 Mol Ethylenoxid an einen Oleyl-Cetylalkohol wird ausgehend von einem Fettalkohol hergestellt, der durch katalytische Hydrierung eines Oleins oder dessen Methylesters zum entsprechenden ungesättigten Alkohol erhältlich ist. Solche Oleyl-Cetylalkohol-Gemische sind, z.B. unter dem Warenzeichen HD-Ocenol®, gekennzeichnet durch den Jodzahlbereich, im Handel erhältlich. Besonders gut geeignet für die Herstellung eines für die Erfindung geeigneten Ethoxylats sind solche technischen Oleyl-Cetylalkohol-Gemische mit einer Jodzahl von 45 bis 85.

Die Sulfatierung kann dann entweder mit Schwefeltrioxid oder mit Chlorsulfonsäuren erfolgen, wobei das Sulfatierungsreagens in einem leichten Überschuß von ca. 1,05 bis 1,1 Mol pro Mol des Ethoxylats eingesetzt wird, um einen hohen Sulfatierungsgrad zu erreichen. Die Sulfatierung kann chargenweise oder kontinuierlich nach bekannten Verfahren durchgeführt werden. Der dabei sich bildende Schwefelsäurehalbester wird dann mit einer Mischung aus wässriger Natronlauge und Magnesiumhydroxid neutralisiert. Da im Endprodukt ein hoher Salzgehalt von 1,4 bis 1,6 Mol/kg vorgesehen ist, kann die Neutralisation auch mit wässriger Natronlauge durchgeführt werden und anschließend das Magnesium in Form von 0,1 bis 0,15 Mol Magnesiumsulfat oder Magnesiumchlorid pro kg Waschrohstoff zugesetzt werden.

Schließlich wird das Ölsäurediethanolamid oder das Linolsäurediethanolamid zugesetzt. Als Ölsäurediethanolamid eignen sich auch Diethanolamide von ungesättigten Fettsäuren mit einem hohen Ölsäure-und/oder Linolsäuregehalt wie z.B. Sonnenblumenölfettsäurediethanolamid oder Sojaölfettsäurediethanolamid. Bevorzugt eignen sich die Diethanolamide technischer Oleine, d.h. der bei 20 °C flüssigen Anteile von Talgfettsäure.

Die Ermittlung der zur Herstellung des erfindungsgemäßen Waschrohstoffs noch zuzusetzenden Menge an Natrium-Salz erfolgt am besten durch Bestimmung der nach Neutralisation im Produkt befindlichen $Cl^{(-)}$ und $SO_4^{(-)(-)}$-Jonen. Befinden sich z.B.-xMol/kg $Cl^{(-)}$ und yMol/kg $SO_4^{(-)(-)}$-Jonen in dem Produkt, so müssen zur Einstellung eines Gehaltes von 1,5 Mol Natrium-Jonen z.B. noch 1,5 - (x + 2 y) Mol Natriumchlorid zugesetzt werden.

Der erfindungsgemäße Waschrohstoff zeichnet sich durch ein besonders günstiges Viskositätsverhalten, ein gutes Schäumvermögen und eine gute Augenschleimhautverträglichkeit aus. Besonders vorteilhaft sind diese Eigenschaften, wenn das enthaltene Alkylethersulfat aus sulfatierten Ethoxylaten besteht, die zusammengesetzt sind aus

40 - 45 Gewichtsprozent eines Anlagerungsproduktes von 10 Mol Ethylenoxid an einem $C_{12}$-$C_{18}$-Kokosfettalkoholschnitt.

40 - 50 Gewichtsprozent eines Anlagerungsproduktes von 2 Mol Ethylenoxid an einem $C_{12}$-$C_{14}$-Kokosfettalkoholschnitt und

8 - 12 Gewichtsprozent eines Anlagerungsproduktes von 2 Mol Ethylenoxid an ein Oleyl-Cetylalkohol-Gemisch.

Der erfindungsgemäße Waschrohstoff eignet sich vor allem zur Herstellung gut schäumender hautverträglicher kosmetischer Haar-und Körperwaschmittel.

Beispiele

1.1 Ein Gemisch aus 96 kg eines Adduktes von 10 Mol Ethylenoxid an einem Kokosfettalkohol $C_{12}$-$C_{18}$ (54 Gewichtsprozent $C_{12}$, 22 Gewichtsprozent $C_{14}$, 10 Gewichtsprozent $C_{16}$, 12 Gewichtsprozent $C_{18}$), 113 kg eines Adduktes von 2 Mol Ethylenoxid an einem Kokosfettalkohol $C_{12}$-$C_{14}$ (73 Gewichtsprozent $C_{12}$, 27 Gewichtsprozent $C_{14}$) und 24,5 kg eines Adduktes von 2 Mol Ethylenoxid an einem Oleyl-Cetyl-Alkohol (65 Gewichtsprozent $C_{18}$, 30 Gewichtsprozent $C_{16}$, 5 Gewichtsprozent $C_{14}$, Jodzahl 50) wurde mit 74,4 kg Chlorsulfonsäure bei einer Temperatur von 10 bis 20 °C in einem kontinuierlichen Reaktor zum Schwefelsäurehalbester umgesetzt und anschließend mit 55,5 kg einer 50 %igen wässrigen Natriumhydroxidlösung neutralisiert. Das dabei erhaltene Alkylethersulfat hatte einen Aniontensid-Gehalt von 0,592 Mol/kg (bestimmt durch Zweiphasentitration nach DGF-Einheitsmethode H-III-10).

1.2 Zu 880 g des Ethersulfats nach 1.1 wurden 28,2 g Mg $SO_4 \cdot 7H_2O$ (0,114 Mol), 20 g Ölsäurediethanolamid, 4,6 g Trinatriumcitrat, 65 g Natriumchlorid (1,1 Mol) und 2,2 kg Wasser gegeben. Es wurde ein flüssiger Waschrohstoff mit einem Aniontensid-Gehalt von 0,59 Mol/kg entsprechend ca. 28 Gewichtsprozent (berechnet mit einem mittleren Molekulargewicht der Aniontenside von 475) erhalten.

2. Prüfung des Viskositätsverhaltens

2.1 Viskositäts-Konzentrations-Verlauf

| Konzentration (Aniontensidgehalt) | Viskosität 20 °C (Höppler Kugelfall-Viskosimeter) |
|---|---|
| 0,59 Mol/kg | 7 500 mPa s |
| 0,54 Mol/kg | 21 500 mPa s |
| 0,49 Mol/kg | 31 500 mPa s |
| 0,44 Mol/kg | 23 200 mPa s |
| 0,39 Mol/kg | 7 000 mPa s |

2.2 Verdickbarkeit durch NaCl-Zusatz

| Konzentration (Aniontensidgehalt) | NaCl-Zusatz (Gew.%) | Viskosität 20 °C (Höppler Kugelfall-Viskosimeter) |
|---|---|---|
| 0,2 Mol/kg | – | 300 mPa s |
| 0,2 Mol/kg | 6 | 1 200 mPa s |
| 0,2 Mol/kg | 7 | 5 600 mPa s |
| 0,2 Mol/kg | 8 | 8 000 mPa s |
| 0,2 Mol/kg | 9 | 12 000 mPa s |

**Ansprüche**

1. Kosmetischer Waschrohstoff in Form eines bei 20 °C pumpbaren wässrigen Konzentrates, gekennzeichnet durch einen Gehalt von

0,4 - 0,6 Mol/kg eines Alkylethersulfatgemisches aus sulfatierten Ethoxylaten, die zusammengesetzt sind aus

35 - 45 Gewichtsprozent eines Anlagerungsproduktes von 8 bis 12 Mol Ethylenoxid an einen im wesentlichen linearen $C_{12}$-$C_{18}$-Fettalkoholschnitt

40 - 60 Gewichtsprozent eines Anlagerungsproduktes von 1 bis 3 Mol Ethylenoxid an einen im wesentlichen linearen $C_{12}$-$C_{14}$-Fettalkoholschnitt

0 - 20 Gewichtsprozent eines Anlagerungsproduktes von 1 bis 3 Mol Ethylenoxid an ein Oleyl-Cetylalkohol-Gemisch, wovon 0,2 bis 0,3 Mol als Magnesiumsalz und der Rest als Natriumsalz vorliegt,

0,03 - 0,08 Mol/kg Ölsäure und/oder Linolsäurediethanolamid und

1,4 - 1,6 Mol/kg Natrium-Ionen in Form von Natriumchlorid und/oder Natriumsulfat.

2. Kosmetischer Waschrohstoff gemäß Anspruch 1, dadurch gekennzeichnet, daß die sulfatierten Ethoxylate zusammengesetzt sind aus

40 - 45 Gewichtsprozent eines Anlagerungsproduktes von 10 Mol Ethylenoxid an einem $C_{12}C_{18}$-Kokosfettalkoholschnitt

45 - 50 Gewichtsprozent eines Anlagerungsproduktes von 2 Mol Ethylenoxid an einen $C_{12}$-$C_{14}$-Kokosfettalkoholschnitt

8 - 12 Gewichtsprozent eines Anlagerungsproduktes von 2 Mol Ethylenoxid an ein Oleyl-Cetylalkohol-Gemisch.

3. Verwendung des kosmetischen Waschrohstoffs zur Herstellung stark schäumender, hautverträglicher kosmetischer Haar-und Körperwaschmittel.